# EUROPEAN PATENT APPLICATION

(11) **EP 2 204 147 A1**
(43) Date of publication of application: **07.07.2010**
(21) Application number: 09000069.6
(22) Date of filing: 06.01.2009
(51) Int. Cl.: A61F 5/02

(54) **Waist protective device**

(71) Applicant: Rao, Yi-Tung, Yuanlin Town Chang hua (TW)
(72) Inventor: Rao, Yi-Tung, Yuanlin Town Chang hua (TW)
(74) Representative: Reichel, Wolfgang

(57) **Abstract**

A waist protective device includes a first support member (10), a second support member (20) spaced from the first support member, two fixing seats (21) each mounted on the second support member, two threaded rods (31) each secured on a respective one of the two fixing seats, two mounting seats (11) each mounted on the first support member, and two rotation members (32) each rotatably mounted on a respective one of the two mounting seats and each having an inner portion provided with an inner threading (321) screwed onto a respective one of the two threaded rods. Thus, the second support member is movable upward relative to the first support member to change the relative position between the second support member and the first support member.

## Description

The present invention relates to a protective device and, more particularly, to a waist protective device to provide an optimum support effect to a user's waist.

A conventional waist protective belt is mounted on a user's waist to provide a tightening effect to the user's waist so as to reinforce the support force of the user's body and to reduce the pressure applied on the user's lumbar vertebra. The waist protective belt may be made of a soft material or a hard material. However, the conventional waist protective belt has a fixed size that cannot be adjusted according to the user's practical requirement. In addition, the conventional waist protective belt only has a covering effect and cannot support the weight of the user's upper body efficiently. Further, the conventional waist protective belt cannot be expanded to stretch the user's waist and cannot reduce the pressure applied on the user's lumbar vertebra efficiently.

In accordance with the present invention, there is provided a waist protective device, comprising a first support member, a second support member spaced from the first support member, two fixing seats each mounted on the second support member, two threaded rods each secured on a respective one of the two fixing seats, two mounting seats each mounted on the first support member, and two rotation members each rotatably mounted on a respective one of the two mounting seats and each having an inner portion provided with an inner threading screwed onto a respective one of the two threaded rods to drive and move the respective threaded rod relative to each of the two rotation members by rotation of each of the two rotation members relative to the respective threaded rod so as to move each of the two fixing seats relative to a respective one of the two mounting seats and to move the second support member relative to the first support member.

The primary objective of the present invention is to provide a waist protective device to provide an optimum support effect to a user's waist.

Another objective of the present invention is to provide a waist protective device, wherein the second support member is movable upward relative to the first support member to change the relative position between the second support member and the first support member according to the user's practical requirement so as to provide the optimum support effect to the user's waist.

A further objective of the present invention is to provide a waist protective device, wherein the first support member and the second support member are located between the two threaded rods, so that the two threaded rods and each of the first support member and the second support member form a three-point support to the user's waist so as to increase the support effect to the user's waist and to decrease the load applied on the user's waist.

A further objective of the present invention is to provide a waist protective device, wherein the second support member is movable upward relative to the first support member by the upward movement of the two threaded rods so as to stretch the user's waist smoothly and stably, thereby providing a comfortable sensation to the user.

Further benefits and advantages of the present invention will become apparent after a careful reading of the detailed description with appropriate reference to the accompanying drawings.

In the drawings:
Fig. 1 is a perspective view of a waist protective device in accordance with the preferred embodiment of the present invention.
Fig. 2 is a partially exploded perspective view of the waist protective device as shown in Fig. 1.
Fig. 3 is a partially perspective cross-sectional assembly view of the waist protective device as shown in Fig. 2.
Fig. 4 is a schematic operational view of the waist protective device as shown in Fig. 3.
Fig. 5 is a schematic operational view of the waist protective device as shown in Fig. 1.
Fig. 6 is a schematic top view of the waist protective device as shown in Fig. 5.

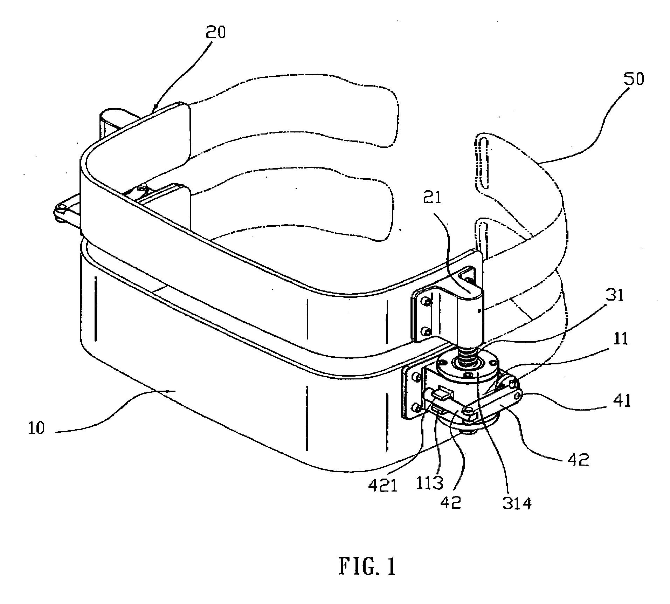

Referring to the drawings and initially to Figs. 1-3, a waist protective device in accordance with the preferred embodiment of the present invention comprises a first support member 10, a second support member 20 spaced from the first support member 10, two fixing seats 21 each mounted on the second support member 20, two threaded rods 31 each secured on a respective one of the two fixing seats 21, two mounting seats 11 each mounted on the first support member 10, two rotation members 32 each rotatably mounted on a respective one of the two mounting seats 11 and each having an inner portion provided with an inner threading 321 screwed onto a respective one of the two threaded rods 31 to drive and move the respective threaded rod 31 relative to each of the two rotation members 32 by rotation of each of the two rotation members 32 relative to the respective threaded rod 31 so as to move each of the two fixing seats 21 relative to a respective one of the two mounting seats 11 and to move the second support member 20 relative to the first support member 10.

The first support member 10 has a substantially curved U-shaped profile. The second support member 20 also has a substantially curved U-shaped profile. The first support member 10 and the second support member 20 are located between the two threaded rods 31. Each of the first support member 10 and the second support member 20 has two opposite ends each provided with a connecting strap 50.

The two mounting seats 11 are secured on two opposite sides of the first support member 10. Each of the two mounting seats 11 has an inner portion provided with a mounting hole 111 for mounting the respective rotation member 32 and has a periphery provided with a shaft hole 112 connected to the mounting hole 111. The shaft hole 112 of each of the two mounting seats 11 is perpendicular to the mounting hole 111. Each of the two mounting seats 11 has a side provided with two spaced clamping pieces 113. The mounting hole 111 of each of the two mounting seats 11 is located between the shaft hole 112 and the two clamping pieces 113.

The two fixing seats 21 are secured on two opposite sides of the second support member 20. Each of the two fixing seats 21 has an inner portion provided with a mounting recess 211 for mounting the respective threaded rod 31 and has a periphery provided with a screw bore 210 connected to the mounting recess 211. The screw bore 210 of each of the two fixing seats 21 is perpendicular to the mounting recess 211.

Each of the two threaded rods 31 has a first end provided with an outer threading 312 screwed into the inner threading 321 of the respective rotation member 32 and a second end secured in the mounting recess 211 of the respective fixing seat 21. The second end of each of the two threaded rods 31 has a periphery provided with an annular retaining groove 311.

The waist protective device further comprises two locking screws 212 each extending through a respective one of the two fixing seats 21 and each pressing the second end of a respective one of the two threaded rods 31 to lock the respective threaded rod 31 onto the respective fixing seat 21. Each of the two locking screws 212 is screwed into the screw bore 210 of the respective fixing seat 21 and is locked in the retaining groove 311 of the respective threaded rod 31.

Each of the two rotation members 32 is rotatably mounted in the mounting hole 111 of the respective mounting seat 11 and is supported by two opposite bearings 313 which are mounted in the mounting hole 111 of the respective mounting seat 11.

The waist protective device further comprises two retaining rings 314 secured on two opposite ends of the mounting hole 111 of a respective one of the two mounting seats 11 to retain a respective one of the two rotation members 32 in the mounting hole 111 of the respective mounting seat 11.

Each of the two rotation members 32 has a periphery provided with a wormwheel 322, and the waist protective device further comprises two propeller shafts 41 each rotatably mounted on a respective one of the two mounting seats 11 and each having a periphery provided with a worm 411 meshing with the wormwheel 322 of a respective one of the two rotation members 32 to rotate the respective rotation member 32 by rotation of each of the two propeller shafts 41 relative to the respective rotation member 32, two cranks 42 each having a first end secured on a respective one of the two propeller shafts 41 to rotate the respective propeller shaft 41, and two drive handles 421 each pivotally mounted on a second end of a respective one of the two cranks 42 to drive and move the respective crank 42. Each of the two drive handles 421 is detachably clamped between the two clamping pieces 113 of a respective one of the two mounting seats 11.

Each of the two propeller shafts 41 is perpendicular to the respective rotation member 32. Each of the two propeller shafts 41 is rotatably mounted in the shaft hole 112 of the respective mounting seat 11 and is supported by two opposite bearings 412 which are mounted in the shaft hole 112 of the respective mounting seat 11.

The waist protective device further comprises an end cap 413 secured on an end portion of the shaft hole 112 of a respective one of the two mounting seats 11 to retain a respective one of the two propeller shafts 41 in the shaft hole 112 of the respective mounting seat 11. Each of the two end caps 413 is provided with a through hole 414, and each of the two propeller shafts 41 extends through and protrudes outwardly from the through hole 414 of the respective end cap 413.

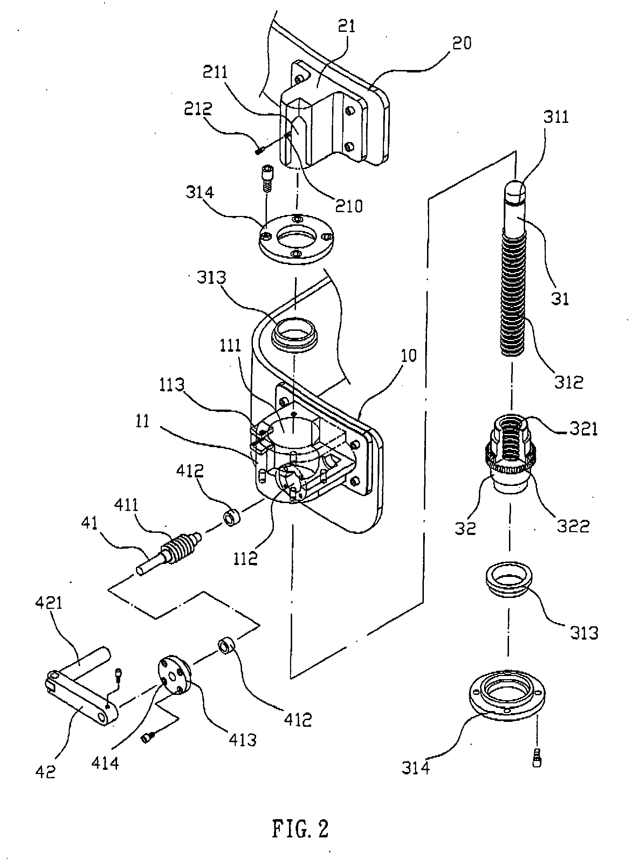

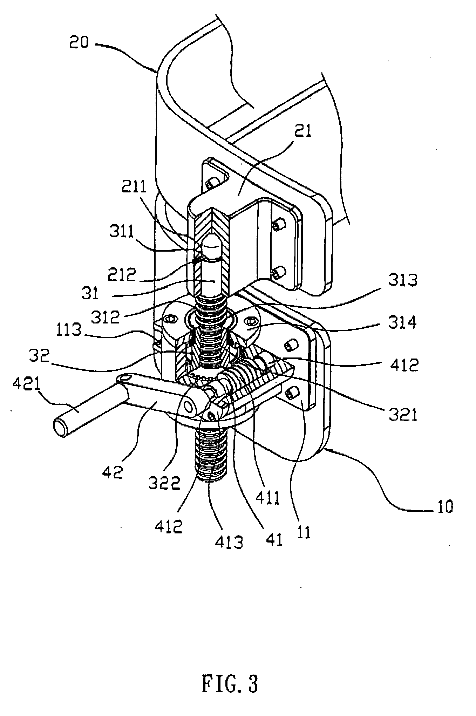

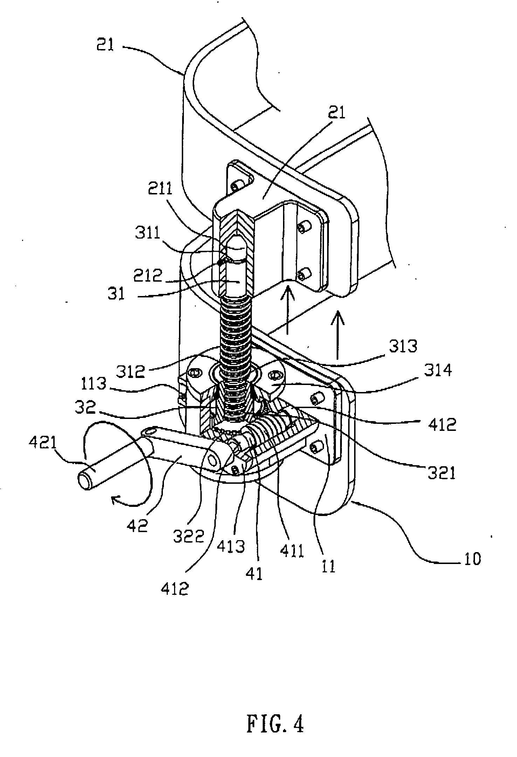

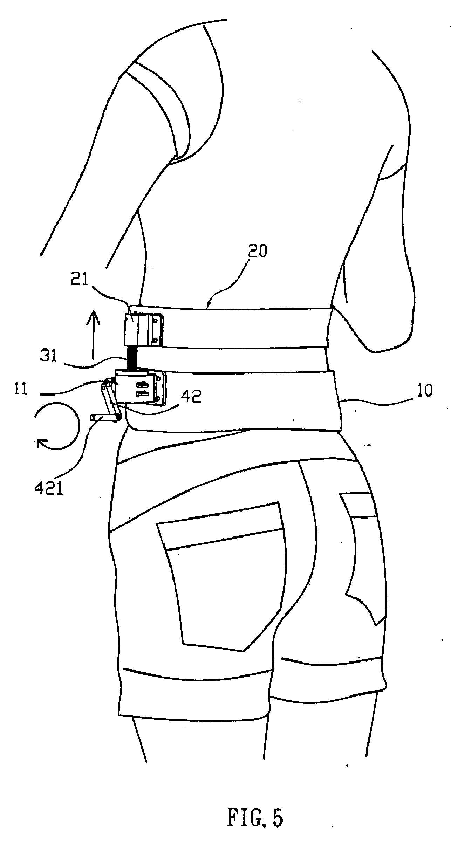

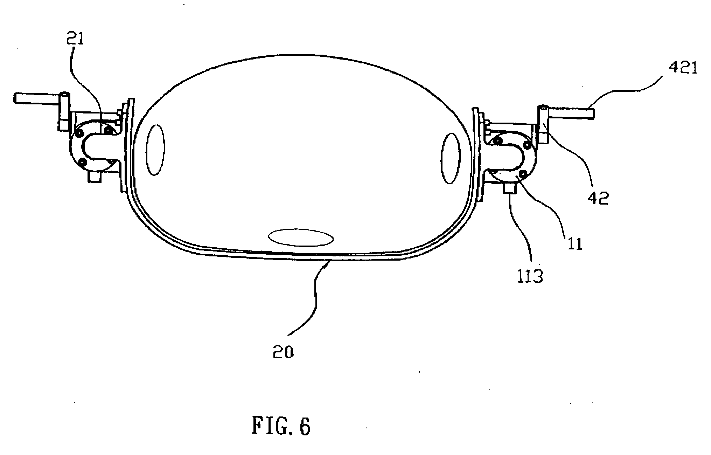

In operation, referring to Figs. 3-6 with reference to Figs. 1 and 2, when the waist protective device is mounted on a user's waist by the connecting straps 50, the user's waist is encompassed by the first support member 10 and the second support member 20. Then, each of the two drive handles 421 is removed from the two clamping pieces 113 of the respective mounting seat 11 and is pivoted outwardly as shown in Fig. 6. Then, each of the two drive handles 421 is moved to rotate the respective crank 42 which rotates the respective propeller shaft 41 which rotates the respective rotation member 32 relative to the respective threaded rod 31. At this time, each of the two threaded rods 31 is secured on the respective fixing seat 21, and the outer threading 312 of each of the two threaded rods 31 is screwed into the inner threading 321 of the respective rotation member 32, so that each of the two threaded rods 31 is moved relative to the respective rotation member 32 as shown in Fig. 4 by rotation of the respective rotation member 32 relative to each of the two threaded rods 31 so as to move the second support member 20 relative to the first support member 10 as shown in Fig. 5 so as to change the relative position between the second support member 20 and the first support member 10.

Accordingly, the second support member 20 is movable upward relative to the first support member 10 to change the relative position between the second support member 20 and the first support member 10 according to the user's practical requirement so as to provide the optimum support effect to the user's waist. In addition, the first support member 10 and the second support member 20 are located between the two threaded rods 31, so that the two threaded rods 31 and each of the first support member 10 and the second support member 20 form a three-point support to the user's waist so as to increase the support effect to the user's waist and to decrease the load applied on the user's waist. Further, the second support member 20 is movable upward relative to the first support member 10 by the upward movement of the two threaded rods 31 so as to stretch the user's waist smoothly and stably, thereby providing a comfortable sensation to the user.

Although the invention has been explained in relation to its preferred embodiment(s) as mentioned above, it is to be understood that many other possible modifications and variations can be made without departing from the scope of the present invention. It is, therefore, contemplated that the appended claim or claims will cover such modifications and variations that fall within the true scope of the invention.

## Claims

1. A waist protective device, comprising:
a first support member (10);
a second support member (20) spaced from the first support member;
two fixing seats (21) each mounted on the second support member;
two threaded rods (31) each secured on a respective one of the two fixing seats;
two mounting seats (11) each mounted on the first support member;
two rotation members (32) each rotatably mounted on a respective one of the two mounting seats and each having an inner portion provided with an inner threading (321) screwed onto a respective one of the two threaded rods to drive and move the respective threaded rod relative to each of the two rotation members by rotation of each of the two rotation members relative to the respective threaded rod so as to move each of the two fixing seats relative to a respective one of the two mounting seats and to move the second support member relative to the first support member.

2. The waist protective device of claim 1, wherein
each of the two mounting seats has an inner portion provided with a mounting hole (111) for mounting the respective rotation member;
each of the two rotation members is rotatably mounted in the mounting hole of the respective mounting seat.

3. The waist protective device of claim 1, wherein each of the two fixing seats has an inner portion provided with a mounting recess (211) for mounting the respective threaded rod.

4. The waist protective device of claim 3, wherein each of the two threaded rods has a first end provided with an outer threading (312) screwed into the inner threading of the respective rotation member and a second end secured in the mounting recess of the respective fixing seat.

5. The waist protective device of claim 4, further comprising:
two locking screws (212) each extending through a respective one of the two fixing seats and each pressing the second end of a respective one of the two threaded rods to lock the respective threaded rod onto the respective fixing seat.

6. The waist protective device of claim 5, wherein
each of the two fixing seats has a periphery provided with a screw bore (210) connected to the mounting recess;
the second end of each of the two threaded rods has a periphery provided with an annular retaining groove (311);
each of the two locking screws is screwed into the screw bore of the respective fixing seat and is locked in the retaining groove of the respective threaded rod.

7. The waist protective device of claim 2, wherein each of the two rotation members is supported by two opposite bearings (313) which are mounted in the mounting hole of the respective mounting seat.

8. The waist protective device of claim 7, further comprising:
two retaining rings (314) secured on two opposite ends of the mounting hole of a respective one of the two mounting seats to retain a respective one of the two rotation members in the mounting hole of the respective mounting seat.

9. The waist protective device of claim 2, wherein
each of the two rotation members has a periphery provided with a wormwheel (322);
the waist protective device further comprises:
two propeller shafts (41) each rotatably mounted on a respective one of the two mounting seats and each having a periphery provided with a worm (411) meshing with the wormwheel of a respective one of the two rotation members to rotate the respective rotation member by rotation of each of the two propeller shafts relative to the respective rotation member.

10. The waist protective device of claim 9, further comprising:
two cranks (42) each having a first end secured on a respective one of the two propeller shafts to rotate the respective propeller shaft;
two drive handles (421) each pivotally mounted on a second end of a respective one of the two cranks to drive and move the respective crank.

11. The waist protective device of claim 9, wherein
each of the two mounting seats has a periphery provided with a shaft hole (112) connected to the mounting hole;
each of the two propeller shafts is rotatably mounted in the shaft hole of the respective mounting seat.

12. The waist protective device of claim 11, wherein each of the two propeller shafts is supported by two opposite bearings (412) which are mounted in the shaft hole of the respective mounting seat.

13. The waist protective device of claim 12, further comprising:
an end cap (413) secured on an end portion of the shaft hole of a respective one of the two mounting seats to retain a respective one of the two propeller shafts in the shaft hole of the respective mounting seat.

14. The waist protective device of claim 10, wherein
each of the two mounting seats has a side provided with two spaced clamping pieces (113);
each of the two drive handles is detachably clamped between the two clamping pieces of a respective one of the two mounting seats.

15. The waist protective device of claim 1, wherein
the first support member and the second support member are located between the two threaded rods;
the two fixing seats are secured on two opposite sides of the second support member;
the two mounting seats are secured on two opposite sides of the first support member.
